# EUROPÄISCHE PATENTSCHRIFT

(11) **EP 2 644 266 B1**
(45) Veröffentlichungstag und Bekanntmachung des Hinweises auf die Patenterteilung: **30.05.2018**
(21) Anmeldenummer: 13001269.3
(22) Anmeldetag: 13.03.2013
(51) Int. Cl.: B01J 20/02, B01J 20/24, B01J 20/28, B01J 20/30, C02F 1/28, C02F 1/62, C02F 1/68, C02F 101/00, C02F 101/20, B01D 53/02, D01F 1/10, D01F 1/02, D01F 2/00, D01F 2/02, A61L 26/00

(54) **Lyocell-Celluloseformkörper zur selektiven Bindung von einwertigen Schwermetallionen, insbesondere von Thallium- und Caesiumionen und deren radioaktiven Isotopen**
Lyocell cellulose mould for the selective binding of monovalent heavy metal ions, in particular of thallium and caesium ions and their radioactive isotopes
Corps de formage cellulosique en lyocell pour la liaison sélective d'ions de métal lourd monovalents, notamment d'ions de thallium et césium et leurs isotopes radioactifs

(30) Priorität: 26.03.2012 DE 102012005947
(43) Veröffentlichungstag der Anmeldung: 02.10.2013
(73) Patentinhaber: Thüringisches Institut Für Textil- Und Kunststoff- Forschung E.V., 07407 Rudolstadt (DE)
(72) Erfinder: Krieg, Marcus, Dr., 99423 Weimar (DE); Sellin, Martin, Dr., 07768 Altenberga (DE); Mooz, Michael, 07422 Saalfelder Höhe (DE)
(74) Vertreter: Plate, Jürgen

(56) Entgegenhaltungen:
- EP-A1- 0 575 612
- DE-C- 848 685
- KAWAMURA S ET AL: "A rapid separation of sodium, potassium, rubidium and caesium by thin layer chromatography on zinc ferrocyanide", JOURNAL OF CHROMATOGRAPHY, ELSEVIER SCIENCE PUBLISHERS B.V, NL, Bd. 26, 1967, Seiten 557-560, XP026731210, ISSN: 0021-9673, DOI: 10.1016/S0021-9673(01)98929-0 [gefunden am 1967-01-01]
- REMEZ V P ET AL: "The Rapid Determination of Caesium Radionuclides in Water Systems Using Composite Sorbents", APPLIED RADIATION AND ISOTOPES, ELSEVIER, OXFORD, GB, Bd. 47, Nr. 9-10, 10. September 1996 (1996-09-10), Seiten 885-886, XP004068593, ISSN: 0969-8043, DOI: 10.1016/S0969-8043(96)00080-2

## Beschreibung

Die Erfindung betrifft cellulosische Formkörper, die nach dem Lyocell-Verfahren hergestellt und in deren CelluloseMatrix Partikel homogen verteilt sind.

Es ist bekannt, dass die Verbindungen aus der Gruppe der Hexacyanoferrate eingesetzt werden um unterschiedliche Isotope von Schwermetallionen der Elemente Cäsium, Strontium, Thallium, Cadmium und Blei aus wässrigen Systemen zu entfernen. Dabei ist es vorteilhaft, wenn die eingesetzten Hexacyanoferrate in einem Trägermaterial so fixiert werden, dass ein Übergang in die das Material umgebende wässrige oder feuchte gasförmige Phase verhindert wird. Bekannte Trägermaterialien für Hexacyanoferrate sind poröse Materialien, wie Ionenaustauscherharze, Sägemehl, Holzstoffe, Faserpulpmaterialien, Papier, natürliche Fasern oder Celluloseregeneratfasern. Die Anreicherung erfolgt dabei stets nur oberflächlich. Die Aufnahmekapazität solcher Trägermaterialien für Hexacyanoferrate ist durch deren Oberfläche und Porenstruktur begrenzt.

Es ist bekannt, dass die Verbindungen aus der Gruppe der Hexacyanoferrate eingesetzt werden um Schwermetallionen der Elemente Cäsium, Strontium, Thallium, Cadmium und Blei aus wässrigen Systemen zu entfernen. Hexacyanoferrate binden die genannten Schwermetallionen durch Ionenaustausch und Einbau in ihr Ionengitter. Die Gruppe der Hexacyanoferrate bildet sich aus elektrisch neutralen Verbindungen, die aus dem negativ geladenen Hexacyanoferrat-anion und positiv geladenen Gegenionen, vorzugsweise Metallkationen zusammengesetzt sind. Aus dieser Gruppe werden vorzugsweise in Wasser schwerlösliche Vertreter verwendet. Typische Beispiele sind Berliner Blau Fe₄[Fe(CN)₆]₃ oder AₓB_{y}[Fe(CN)₆] (A = NH₄/Li/Na/K, B = Fe/Ni/Co/Cu/Ti/Cr).

Das Patent DE 3735304 und der IAEA Report "The use of Prussian Blue to reduce radiocaesium contamination of milk and meat produced on territories affected by the Chernobyl accident" vom Februar 1997 beschreiben den Einsatz von Hexacyanoferraten zur Bindung von Tl⁺ und Cs⁺-Isotopen in der Humanmedizin und Tiermedizin. Die oral applizierten Hexacyanoferrate werden vom Körper wieder ausgeschieden. Bei diesen Anwendungen ist keine Abtrennung oder Trägerung notwendig, so dass reine Hexacyanoferrate zum Einsatz kommen können. Bei den Anwendungen von Hexacyanoferraten zur Entfernung von Schwermetallionen aus wässrigen Systemen hingegen besteht der Wunsch diese vollständig vom System abzutrennen. Die unlöslichen mikrokristallinen Hexacyanoferrate wie NH₄Fe[Fe(CN)₆] oder Fe₄[Fe(CN)₆]₃ haben die die Eigenschaft kollodiale Lösungen in Wasser zu bilden. Kolloidpartikelgrößen zwischen 1 und 100 µm und kleiner 1 µm begrenzen den Einsatz reiner Hexacyanoferrate in Filterschichten. Filterschichten aus derartigen Hexacyanoferraten sind mit großen Druckverlusten über die Länge der Filterschüttung verbunden. Die kollodiale Löslichkeit führt weiterhin zu einem Austrag des Hexacynoferrates auch durch Barrieren mit Porengrößen von kleiner als 0,1 µm.

In der EP 0 575 612 A1 sind poröse, partikelförmige oder faserartige Trägermaterialien beschrieben, die mit einer Suspension von Hexacyanoferraten behandelt sind. Die so behandelten Trägermaterialien binden insbesondere radioaktive Cäsium-, Rubidium- und Strontiumionen. Bei dem Versuch, Cellulosefasern, insbesondere Lyocell-Cellulosefasern, mit Hexacyanoferraten zu beladen, zeigten sich mehrere Nachteile. So war die Menge an Hexacyanoferraten, mit denen die Fasern beladen werden konnten, begrenzt. Die Hexacyanoferrat-Partikel zeigten zudem eine geringe Haftung, so dass sie leicht auswaschbar waren. Die unzureichende Haftung der Hexacyanoferrat-Partikel an den Cellulosefasern führte zudem zu Staubentwicklung bei der Verarbeitung. Zudem war die Verteilung der Hexacyanoferrat-Partikel auf den Fasern nicht homogen.

Aus der Publikation von S. Kawamura et al., J. Chromatog. 26 (1967), Seiten 557-560, ist eine Glassplatte zur Dünnschichtchromatographie wässriger Lösungen verschiedener einwertiger Metallionen bekannt, welche eine stationäre Phase aufweist, die aus einer Suspension aus pulverförmiger Cellulose und Zink-Ferrocyanid auf die Glasplatte aufgebracht wird.

Aus der Publikation von V.P. Remez et al., Appl. Radiat. 47 No. 9/10 (1996), Seiten 885-886 ist ein Komopsit-Sorptionsmittel "Anfezh" zur Sorption von Cäsium-Radionukliden aus Wasser bekannt, bei dem ein Eisen-Kalium-Hexacyanoferrat auf einem Celluloseträger aufgebracht ist. Es besteht daher der Wunsch, Hexacyanoferrate wie NH₄Fe[Fe(CN)₆] oder Fe₄[Fe(CN)₆]₃ möglichst ohne Verschlechterung ihrer Sorptionsfähigkeit für bestimmte Schwermetallionen in eine Matrix einzubinden und so die Bildung kollodialer Lösungen zu verhindern. Die Hexacyanoferrate sollen fest in der Matrix eingebunden und homogen verteilt sein. Die entsprechenden Formkörper sollen als Filtermaterial mit hoher Aufnahmekapazität für Schwermetallionen nutzbar sein. Für diese Aufgabe wurden verschiedene Verfahren und Lösungen beschrieben welche jeweils Nachteile aufweisen.

Eine vorgeschlagene Lösung für die Aufgabe ist die Fixierung von Hexacyanoferraten an Ionenaustauschermaterialien. So wird in EP1419009 ein Kompositmaterial auf der Basis eines Trägermaterials mit einer Beschichtung aus Ionenaustauschermaterial zur ionischen Fixierung verwendet. In RU2033240 ist weiterhin die Bindung von Hexacyanoferrat an Viskosefasern mit Ionenaustauscherfunktionalisierung beschrieben. Auch poröse Naturstoffe wie Sägemehl sind nach EP575612 in der Lage Hexacyanoferrate aufzunehmen und damit zu binden. Bei einer ionischen Fixierung ist eine Verdrängung vom Ionenaustauscher durch andere Ionen möglich. Oberflächlich beschichtete poröse Trägermaterialien hingegen weisen bekanntermaßen Grenzen bezüglich der Haftung und mechanischen Stabilität auf.
Ein weiterer Lösungsansatz verfolgt die Idee unlösliche Hexacyanoferrate in den Poren von porösen Trägermaterialien zu erzeugen und damit zu fixieren. In US5601722 kommt dieses Verfahren zum Einsatz. Nachteilig ist hierbei, dass das Verfahren mehrere Einzelschritte für die Herstellung benötigt und nur eine rein mechanische Bindung an den Träger vorliegt. Des Weiteren ist die Kinetik der Sorption von Schwermetall-ionen durch die Zugänglichkeit der inneren Porenstruktur begrenzt.

Es besteht daher nach wie vor ein Bedarf an CelluloseFormkörpern zur selektiven Bindung von einwertigen Schwermetallionen, insbesondere von Thallium- und Cäsiumionen und deren radioaktiven Isotopen.

Überraschenderweise wurde gefunden, dass Hexacyanoferrate wie NH₄Fe[Fe(CN)₆] oder Fe₄[Fe(CN)₆]₃ in cellulosische Formkörper direkt integriert werden können und so vor dem Ausbluten geschützt sind. Durch das Herstellungsverfahren wird das verwendete Hexacyanoferrat gleichmäßig im Träger Cellulose verteilt und von diesem vollständig umschlossen. Ein derartiges Kompositmaterial zeigt in wässrigem Medium keine Verringerung der Sorptionsfähigkeit für Schwermetallionen wie Thallium oder Caesium gegenüber dem reinen Hexacyanoferrat. Dies ist durch die Quellbarkeit und Porosität der Cellulosematrix in wässrigen Systemen zu erklären, welche einen guten Zugang der Schwermetallionen an das Hexacyanoferrat gewährleistet.

Das erfindungsgemäße Kompositmaterial stellt eine Lösung für den gewünschten Einsatz zur Bindung von Schwermetallionen in wässrigen Systemen dar. Das Kompositmaterial wird direkt aus dem gewünschten Hexacyanoferrat und dem cellulosischen Träger hergestellt. Es zeichnet sich durch eine homogene Verteilung des Hexacyanoferrates in der Matrix aus. Die Adsorptionsfähigkeit des Hexacyanoferrates wird durch die Einbindung in die Matrix nicht wesentlich vermindert. Durch wässrige Medien tritt keine Freisetzung von Hexacyanoferraten aus der Matrix auf.

Die beschriebene Zusammensetzung, hier bezeichnet als Kompositmaterial, aus Cellulose und Hexacyanoferrat vereinigt dabei eine sichere Trägerung eines aktiven Hexacyanoferrates mit hoher Beladung unter Erhalt der erwünschten Fähigkeit Schwermetalle zu binden.

Das Kompositmaterial zeichnet sich durch eine gleichmäßige Verteilung der Hexacyanoferratpartikel über den gesamten Querschnitt aus. Der Zugang der Schwermetallionen an die Hexacyanoferrate wird durch die Quellbarkeit des cellulosischen Trägermaterials gewährleistet. Ein derartiges Kompositmaterial wird durch Regeneration einer Celluloselösung mit suspendierten Hexacyanoferratpartikeln in Wasser erhalten.
Je nach Applikation ist das Kompositmaterial eine Folie, Faser, Borste, Granulat, Fibrid, Membran, Schaum oder Spinnvlies sowie davon abgeleitete Erscheinungsformen wie Vliesstoff, Gewebe, Gewirk, Gestrick oder Papier.

Die Herstellung von Celluloseformkörpern mit integrierten funktionellen Additiven ist Stand der Technik und lässt sich durch Regeneration von Celluloselösungen oder Cellulosederivaten mit eingemischten Additiven realisieren.

Etablierte Verfahren sind dabei etwa das Viskoseverfahren, das Cuoxamverfahren und das Lyocellverfahren mit tertiären Aminoxiden. Mit dem Trocken-/Naß-Spinnverfahren gemäß der DE 44 26 966 C2 lassen sich Lyocell-Cellulosefäden und - folien mit einem hohen Anteil an Zusatzstoffen herstellen. Die Cellulose wird dabei rein physikalisch in einem geeigneten Lösungsmittel, wie N-Methyl-morpholin-N-oxid-Monohydrat, gelöst. Die Zusatzstoffe werden dann in der Lösung fein verteilt. Die Mischung wird dann zu Fäden oder Folien geformt. In einem Wasserbad wird dann das Lösungsmittel entzogen. Erhalten werden Cellulose-Formkörper mit eingeschlossenen und dadurch fixierten Feststoffen. Die geringe Säurestabilität und Alkalistabilität von Hexacyanoferraten wie NH₄Fe[Fe(CN)₆] oder Fe₄[Fe(CN)₆]₃ schließt diese Herstellungsverfahren allerdings aus. Bei den üblichen Lösemitteln und Fällmedien des Lyocell-Verfahrens und den dabei herrschenden Prozeßbedingungen kommt es zu Unverträglichkeiten zwischen dem Berliner Blau und dem Lösungs- bzw. Fällmedium.

Überraschenderweise wurde gefunden, dass das beschriebene Kompositmaterial mit alternativen Lösungsmitteln für Cellulose erhalten werden kann. Dies gelingt vor allem, wenn für das Lösen und Regenerieren der Cellulose keine sauren oder alkalischen Bedingungen verwendet werden müssen. Dies ist bei der Verwendung von Lösungsmittelsystemen wie Ionischen Flüssigkeiten, DMSO/TBAF, LiCl/DMAc, LiCl/DMF gegeben. Das beschriebene Kompositmaterial läßt sich unter Verwendung von ionischer Flüssigkeit, im Besonderen mit 1-Butyl-3-methylimidazolium-chlorid und Regeneration in reines Wasser erhalten. Verallgemeinert ist eine Herstellung durch das Lösen von Cellulose mit Celluloselösungsmitteln und durch Regeneration in Nichtlösungsmitteln für Cellulose, besonders in Wasser, möglich, solange prozessbedingt nötige Säuren und Basen nicht zu einer Veränderung oder Zerstörung der Hexacyanoferrate führen. Durch das Lyocell-Verfahren mit ausgewählten ionischen Flüssigkeiten als Lösemittel ist es erstmalig gelungen, Berliner Blau in Celluloseregenerat-Fasern homogen zu inkorporieren und Lyocell-Formkörper mit einer hohen Beladung an Hexacyanoferrat-Partikeln herzustellen.
Gegenstand der Erfindung sind demgemäß Lyocell-Celluloseformkörper gemäß Anspruch 1. Das Hexacyanoferrat ist bevorzugt eine ladungsneutrale chemische Verbindung aus Hexacyanoferratanionen und Kationen, bevorzugt Cobalt, Kupfer, Natrium, Kalium oder Ammonium, besonders bevorzugt Eisen, mit Partikelgrößen zwischen 0,001 µm und 100 µm, bevorzugt zwischen 0,1 µm und 50 µm.

Die nach einem solchen Verfahren hergestellten Formkörper sind im folgenden als Lyocell-Cellulose-Formkörper bezeichnet. Im Lyocell-Verfahren werden cellulosische Formkörper ohne Derivatisierung der Cellulose über ein Lösungsspinnverfahren mit organischen Lösemitteln oder Gemischen organischer Lösemittel und Wasser erhalten (Terminologie gemäß BISFA 2009). Der Begriff "Formkörper" bezeichnet im Zusammenhang mit der vorliegenden Erfindung Fasern, Fibride, Vliese, Granulate, Beads, Folien, Folienschläuche, Filamente, Schwämme, Schäume und Borsten, wobei textil verarbeitbare Fasern bevorzugt sind.

Dabei wurde überraschenderweise gefunden, dass die erfindungsgemäßen Lyocell-Cellulose-Formkörper mit homogen über die Matrix verteilten Hexacyanoferrat-Partikeln eine hohe Sorptionskapazität für einwertige Schwermetallionen, insbesondere Thallium- und Cäsiumionen und deren radioaktiven Isotopen aufweisen. Obwohl die Hexacyanoferrat-Partikel vollständig in eine polymere Matrix eingeschlossen sind, wurde die eigentlich zu erwartende Verminderung der Sorptionskapazität nicht beobachtet. Die Sorptionskapazität der erfindungsgemäßen Lyocell-Cellulose-Formkörper liegt in der gleichen Größenordnung wie die Sorptionskapazität einer vergleichbaren Menge an nicht polymergebundenem Hexacyanoferrat-Pulver.

Der beschriebene Lyocell-Cellulose-Formkörper kann unterschiedliche Partikelgrößen der Hexacyanoferrate enthalten. Erfindungsgemäß sind die Dimensionen des Lyocell-Cellulose-Formkörpers größer als die der verwendeten Hexacyanoferratpartikel. Auch Hexacyanoferrate mit einem Durchmesser kleiner als 1 µm werden gleichmäßig in der Matrix verteilt und die Bildung kollodialer Lösungen in angrenzenden wässrigen Phasen verhindert. Je nach Dicke des Lyocell-Cellulose-Formkörpers können Hexacyanoferrat-Kristallite mit Durchmessern von 1 µm bis 100 µm und kleiner als 1 µm verarbeitet werde. Dies ermöglicht eine Verbesserung der Sorptionskinetik für Schwermetallionen mit sinkendem Partikeldurchmesser.

Das verwendete Herstellungsverfahren ermöglicht sehr geringe Gewichtsanteile von < 1% Hexacyanoferrat, bezogen auf das Kompositmaterial, und hohe Gewichtsanteile bis zu 80% Hexacyanoferrat, bezogen auf den Lyocell-Cellulose-Formkörper. Erfindungsgemäß haben die Hexacyanoferrate einen Anteil von 0,1 bis 80 Gew.-%, besonders bevorzugt von 10 bis 50 Gew.-%, an der Gesamtzusammensetzung. Das Herstellungsverfahren kann als einfacher, einstufiger Prozeß gestaltet werden.

Die cellulosische Matrix des beschriebenen Lyocell-Cellulose-Formkörpers kann zu 100% aus Cellulose bestehen. Weiterhin kann sich die cellulosische Matrix aus einer Mischung von mehr als 50 Gew.-% Cellulose mit polymeren Additiven, wie Cellulosederivaten, und partikulären Additiven, wie Ruß, Aktivkohle, Ionenaustauscherharz, anorganischen Pigmenten und Salzen, zusammensetzen.
Das verwendete Herstellungsverfahren ermöglicht die Herstellung unterschiedlicher Erscheinungsformen des Lyocell-Cellulose-Formkörpers. Je nach Applikation ist die direkte Ausformung bevorzugt als Fibrid, Faser, Spinnvlies, Beads, Granulat, Folie, Folienschlauch, Filament, Schwamm oder Borste Stand der Technik. Die Weiterberarbeitung eines der genannten Erscheinungsformen zu Vliesstoff, Gewebe, Gewirk, Gestrick, Papier oder anderen Produkten die den beschriebenen Lyocell-Cellulose-Formkörper in Anteilen von 1 bis 100 % enthalten ist ebenfalls möglich.

Trotz der Einbindung von Hexacyanoferrat in eine cellulosische Matrix ist eine Trennung von Matrix und Hexacyanoferrat möglich. Dies erlaubt ein Recycling der Hexacyanoferrate, die Zuführung der reinen Hexacyanoferrate zur Rückgewinnung der gebundenen Schwermetalle oder eine Gewichtsreduktion beim Deponieren. Dafür kann die cellulosische Matrix durch bekannte Lösungsmittel für Cellulose aufgelöst werden, durch Säuren zersetzt werden, durch chemische Reaktionen in ein Cellulosederivat überführt werden oder durch Kompostierung zersetzt werden.

In den erfindungsgemäßen, nach dem Lyocell-Verfahren hergestellten cellulosischen Formkörpern sind die Hexacyanoferrate in einer Polymermatrix fest verankert und so vor dem Ausbluten in eine wäßrige Lösung geschützt. Durch diese Verankerung läßt sich das Kompositmaterial sehr gut in Filteranwendungen nutzen. Es ermöglicht eine leichte Abtrennung nach Verwendung ohne den Einsatz von Zentrifugen und Filtern. Dies gelingt nur, wenn der Lyocell-Cellulose-Formkörper den Übergang der kollodial wasserlöslichen Hexacyanoferrate in die wäßrige Phase verhindert. Durch die beschriebene Verankerung der Hexacyanoferate ist ihre Fähigkeit, Schwermetallionen zu binden, nicht beeinträchtigt. Die beschriebene Verankerung von Hexacyanoferraten ist besonders für diese Aufgaben geeignet. Die cellulosische Polymermatrix ermöglicht einen guten Zugang der Schwermetallionen zu den aktiven Hexacyanoferraten durch Quellung mit Wasser. Die direkte Ausformung der Ausgangsstoffe zum Lyocell-Cellulose-Formkörper ergibt eine gleichmäßige Verteilung der Hexycyanoferrate über den gesamten Querschnitt. Die Hexacyanoferrate sind weiterhin vollständig von der Polymermatrix umschlossen. Verluste von Hexacyanoferrat durch mechanische Einflüsse oder durch Ablösen von Oberflächen sind somit vermindert.

Der erfindungsgemäße Lyocell-Cellulose-Formkörper zeichnet sich durch seine hohe Variabilität der möglichen Formkörper und damit verbundene Anwendbarkeit aus. Je nach Ausformung als Folie, Faser, Borste, Granulat, Fibrid oder Spinnvlies ergeben sich unterschiedliche Anwendungen. Von besonderem Interesse ist die Verwendung als Filtermaterial in flüssiger und feuchter Umgebung zur Sorption von Schwermetallionen. Dies beinhaltet zum Beispiel die Reinigung von Wasser mit Thallium- und Caesiumkontamination oder die Gewinnung, Abtrennung oder Rückgewinnung dieser Elemente aus wäßrigen Lösungen oder Prozessströmen. Dies beinhaltet auch die Möglichkeit das Kompositmaterial zur Thallium- und Caesiumentgiftung von Organismen zu verwenden. Gegenüber dem reinen Hexacyanoferrat ist mit dem Lyocell-Cellulose-Formkörper eine Modifikation der Wirkstofffreisetzung oder die Möglichkeit der äußerlichen Anwendung mit Wundauflagen gegeben.

Bevorzugt beträgt die Kapazität für die Bindung von Schwermetallionen, insbesondere von Thallium- und Caesiumionen, zwischen 20% und 400%, und im Besonderen zwischen 75% und 200%, bezogen auf das enthaltene Hexacyanoferrat und verglichen mit dem reinen Hexacyanoferrat.
Der Lyocell-Cellulose-Formkörper eignet sich weiterhin für die Filtration von Dämpfen sowie Aerosolen und damit zur Filtration von Gasen und Luft. Die Feuchtigkeitsaufnahme und das Rückhaltevermögen für Feuchtigkeit der cellulosischen Matrix ermöglicht dabei die Aufnahme von Schwermetallkontaminationen auch aus solchen Medien. Damit ist auch die Ausrüstung von persönlicher Schutzkleidung und Atemschutzgeräten mit diesem Lyocell-Cellulose-Formkörper möglich.

Die Erfüllung der Aufgabe des Lyocell-Cellulose-Formkörpers wird neben der Art der genannten Ausformungen auch durch die Weiterverarbeitung dieser Ausformungen ermöglicht. Dies beinhaltet zum Beispiel die Verarbeitung oder Zugabe der Lyocell-Cellulose-Formkörper zu Schüttungen, Mahlungen, Geweben, Gewirken, Gestricken, Papieren oder Vliesen.

Die Erfindung wird nachfolgend anhand von Zeichnungen näher beschrieben; es zeigen;
Fig. 1 einen Querschnitt einer Lyocellfaser beladen mit Hexacyanoferraten nach dem bekannten Verfahren durch Tränken oder Imprägnieren nach EP 0 575 612 A1,
Fig. 2 einen Querschnitt einer Lyocellfaser beladen mit Hexacynoferraten entsprechend dieser Erfindung.

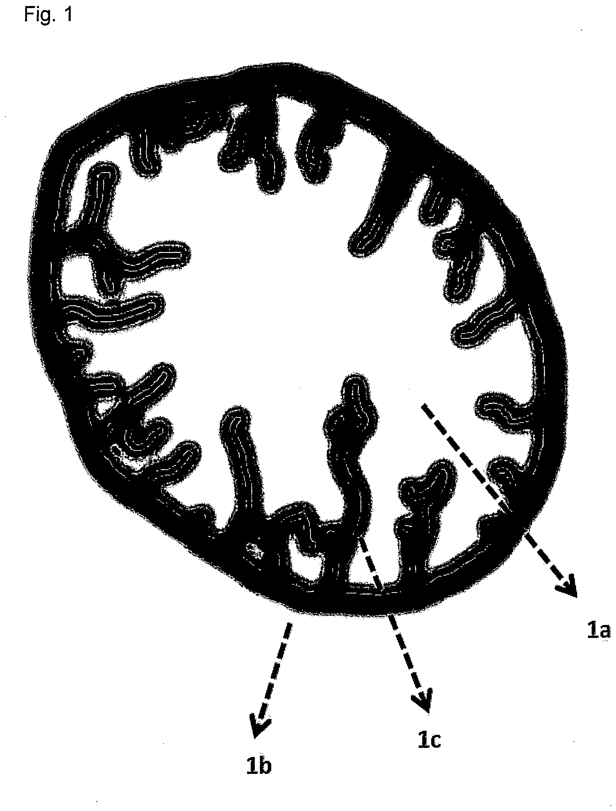

Der in Fig. 1 veranschaulichte Faserquerschnitt besteht aus einer porösen Lyocellmatrix 1a. Diese Lyocellmatrix besitzt an der Oberfläche eine Beschichtung mit Hexacyanoferraten 1b und mit Hexacyanoferraten gefüllte Poren 1c.

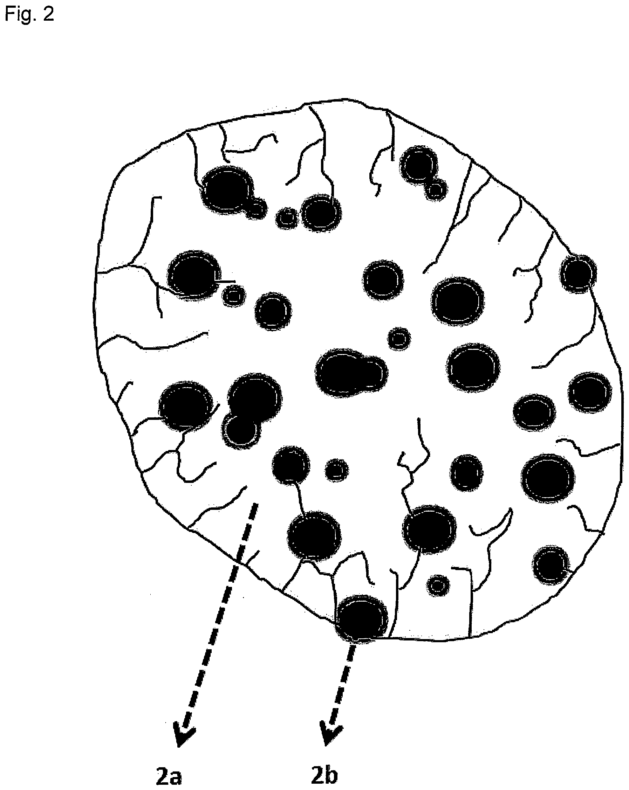

Der in Fig. 2 veranschaulichte Faserquerschnitt besteht aus einer porösen Lyocellmatrix 2a. Diese Lyocellmatrix enthält Hexacyanoferrate 2b. Die Hexacyanoferrate sind über den gesamten Querschnitt gleichmäßig verteilt und werden von der Lyocellmatrix umschlossen.

Die nachfolgenden Beispiele dienen zur Illustration der Erfindung. Prozente sind darin als Gewichtsprozente zu verstehen, soweit nicht anders angegeben oder aus dem Zusammenhang ersichtlich.

### Beispiel 1

Ein zur selektiven Sorption von Thallium und Caesium hergestelltes Hexacyanoferrat Fe₄[Fe(CN)₆]₃ wurde mit Hilfe eines Mörsers pulverisiert und durch ein kleinmaschiges Sieb auf Partikelgrößen kleiner als 40 µm ausgesiebt. Das erhaltene Material mit einer Partikelgröße kleiner als 40 µm (x₉₀ = 31,9 µm, x₅₀ = 10,36 µm) wurde anschließend mit Cellulose zu einer Cellulosefolie mit einem Durchmesser von 100 µm und einer Beladung von 10 Gew.-% Hexacyanoferrat verarbeitet.

Es wurde eine Mischung aus 165 g Wasser, 386 g 1-Butyl-3-methylimidazolium-chlorid, 27 g gemahlener Cellulose mit einem Polymerisationsgrad (DP) von 615 und 3 g des Hexacyanoferrates hergestellt. Diese Mischung wird mit Hilfe eines Ultraturrax homogenisiert und anschließend in einem Planetenrührer unter Scherung und Wärmezufuhr bei einem Vakuum von 10 mbar bei einer Maximaltemperatur von 105°C behandelt. Als Resultat wurde eine homogene Mischung erhalten, welche 9% Cellulose, 1% Hexacyanoferrat, 89,9% 1-Butyl-3-methylimidazolium-chlorid und 0,1% Wasser enthielt.

Diese Mischung wurde mit zu einer dünnen Schicht ausgestrichen und zur Koagulation der Cellulose und zum Auswaschen des Lösungsmittels in einem Wasserbad gelagert. Nach vollständiger Koagulation wurde das Kompositmaterial in Form einer Folie mit einer Dicke von 30 µm erhalten. Diese intensivblaue Folie mit 10 Gew.-% Hexacyanoferrat wurde auf einem Spannrahmen getrocknet. Die Restfeuchte des Materials beträgt 10 Gew.-%.

### Beispiel 2

Ein zur selektiven Sorption von Thallium und Caesium hergestelltes Hexacyanoferrat Fe₄[Fe(CN)₆]₃ wurde mit Hilfe einer Gegenstrahlmühle auf eine Partikelgröße kleiner als 7 µm gemahlen (x₉₀ = 4,66 µm, x₅₀ = 2,88 µm). Das erhaltene Material wurde anschließend mit Cellulose zu Stapelfasern der Feinheit 3 dtex und einer Beladung von 10 Gew.-% Hexacyanoferrat absolut verarbeitet.

Aus 1654 g Wasser, 3860 g 1-Butyl-3-methylimidazolium-chlorid, 270 g gemahlener Cellulose mit einem Polymerisationsgrad (DP) von 615 und 30 g gemahlenem Hexacyanoferrat wurde analog zu Beispiel 1 eine homogene Mischung hergestellt. Die Ausformung dieser Mischung zu Stapelfasern erfolgte analog der Lyocelltechnologie zur Erzeugung von Cellulosestapelfasern.

Das erhaltene Faser-Kompositmaterial wurde auf eine Länge von 38 mm geschnitten und bei 50°C bis zu einer Restfeuchte von 10 Gew.-% absolut getrocknet.

Parameter der erhaltenen Stapelfasern aus Beispiel 2:

| | |
|---|---|
| Feinheit [dtex] | 3,23 |
| Stapelfaserlänge [mm] | 38 |
| Feinheitsbezogene Reißkraft [cN/tex] | 37,7 |
| Feinheitsbezogene Schlingenreißkraft [cN/tex] | 5,24 |
| Reißdehnung [%] | 10,8 |

### Beispiel 3

Eignung für die Bindung für Thallium und Caesium in Filtrationsanwendungen:
100 mL einer Testlösung mit 0,2 mol/L Natriumchlorid und 0,1 mol/L Thallium(I)-nitrat oder 0,1 mol/L Caesium(I)-sulfat in entionisiertem Wasser wurden mit 1 g des Kompositmaterials oder 0,1 g des eingesetzten Hexacyanoferrates für 24 Stunden unter Rühren in Kontakt gebracht. Die Sorption von Thallium-ionen oder Caesiumionen durch das Material wurde durch Bestimmung der Konzentration dieser Elemente nach Abtrennung des Materials in der Testlösung mittels ICP-OES bestimmt. Das Bindungsvermögen wird in Gramm aufgenommenem Ion (Thallium, Caesium) bezogen auf das eingewogene Material angegeben und basiert jeweils auf einer Dreifachbestimmung.

| Material | Gew.-% Hexacyanoferrat | Sorption Tl⁺ g/g | Sorption Cs⁺ g/g |
|---|---|---|---|
| Beispiel 1 Fe₄[Fe(CN)₆]₃ < 40 µm | 100 | 0,302 | 0,279 |
| Beispiel 1 Folie | 10 | 0,062 | 0,021 |
| Beispiel 2 Fe₄[Fe(CN)₆]₃ < 7 µm | 100 | 0,291 | 0,297 |
| Beispiel 2 Faser | 10 | 0,032 | 0,027 |

Die Kapazität für die Bindung von Thallium und Caesium wurde demnach durch die Einarbeitung in die cellulosische Matrix nur im geringen Maße beeinflusst. In einigen Fällen war eine Steigerung des Bindevermögens zu beobachten. Die Sorption von Caesium des Hexacyanoferrates betrug sowohl in der Faser als auch in der Folie noch 75-90% gegenüber dem reinen Hexacyanoferrat ohne Matrix. Eine Folie mit dem Hexacyanoferrat < 7 µm aus Beispiel 2, hergestellt analog zu Beispiel 1, erreichte sogar 110% der Caesiumbindung gegenüber dem eingesetzten Hexacyanoferrat. Die Bindung von Thallium konnte durch die Einarbeitung in die cellulosische Matrix gesteigert werden. In Beispiel 1 verdoppelte sich die Kapazität für Thallium, verglichen mit dem eingesetzten reinen Hexacyanoferrat. Eine Steigerung der Kapazität für die selektive Bindung von Thallium oder Caesium ist ein besonderer Vorteil dieses Lyocell-Cellulose-Formkörpers. Die cellulosische Matrix kann einen positiven Effekt auf die Gesamtbilanz der Thallium oder Caesiumbindung ausüben.

Der beschriebene Lyocell-Cellulose-Formkörper (Faser, Folie) konnte ohne Filtration oder Zentrifugieren vollständig aus der Testlösung entnommen werden. Das eingesetzte Hexacyanoferrat mit den Partikelgrößen < 40 µm und < 7 µm konnte nur mit Hilfe von mehrfachem Zentrifugieren und Filtrieren mit Filtermaterialien der Porengröße 0,02 µm von der Testlösung abgetrennt werden.

## Patentansprüche

1. Lyocell-Celluloseformkörper zur Bindung von Schwermetallionen und deren radioaktiven Isotopen, **dadurch gekennzeichnet, dass** ein oder mehrere Hexacyanoferrate in eine cellulosische Matrix eingebunden, von dieser vollständig umschlossen, und homogen über den gesamten Querschnitt der cellulosischen Matrix verteilt sind, wobei die Hexacyanoferrate einen Anteil von 0,1 bis 80 Gew.-% am Gesamtgewicht des Celluloseformkörpers haben.

2. Celluloseformkörper nach Anspruch 1, **dadurch gekennzeichnet, dass** das Hexacyanoferrat eine ladungsneutrale chemische Verbindung aus Hexacyanoferratanionen und Kationen, bevorzugt Cobalt, Kupfer, Natrium, Kalium oder Ammonium, besonders bevorzugt Eisen, mit Partikelgrößen zwischen 0,001 µm und 100 µm, bevorzugt zwischen 0,1 µm und 50 µm, ist, wobei die Partikelgröße durch Sieben bestimmt wird.

3. Celluloseformkörper nach Anspruch 1, bei der die cellulosische Matrix entweder aus reiner Cellulose oder aus einer Mischung von mehr als 50% Gew.-% Cellulose mit polymeren Additiven, bevorzugt Cellulosederivaten, und/oder partikulären Additiven, bevorzugt Ruß, Aktivkohle, Ionenaustauscherharzen, anorganischen Pigmenten und/oder Salzen, besteht.

4. Celluloseformkörper nach Anspruch 1, **dadurch gekennzeichnet, dass** die Hexacyanoferrate einen Anteil von 10 bis 50 Gew.-% an dessen Gesamtgewicht haben.

5. Celluloseformkörper nach Anspruch 1 **dadurch gekennzeichnet, dass** er ein Fibrid, eine Faser, ein Vlies, ein Granulat, Bead, eine Folie, ein Folienschlauch, ein Filament, ein Schwamm oder eine Borste ist.

6. Verwendung des Celluloseformkörpers nach einem oder mehreren der Ansprüche 1 bis 5 zur Sorption von einwertigen Metallionen, insbesondere der Elemente Thallium und Caesium und deren Isotope, in wässrigen oder feuchten Systemen.

7. Verwendung eines Material nach Anspruch 6 für Wasseraufbereitung und Wasserdekontamination, Metallgewinnung, Wundbehandlung mit Wundauflagen, Luftfiltration, Gasfiltration und Schutzkleidung.

## Claims

1. A formed lyocell cellulose article for binding heavy metal ions and radioactive isotopes thereof, wherein one or more hexacyanoferrates are incorporated in a cellulosic matrix, and, fully enclosed by said matrix, distributed homogeneously throughout the entire cross section of the cellulosic matrix, where the hexacyanoferrates comprise from 0.1 to 80 wt% of the overall weight of the formed cellulose article.

2. The formed cellulose article as claimed in claim 1, wherein the hexacyanoferrate is a neutral-charge chemical compound composed of hexacyanoferrate anions and cations, preferably cobalt, copper, sodium, potassium or ammonium and more preferably iron, having particle sizes between 0.001 µm and 100 µm and preferably between 0.1 µm and 50 µm, where the particle size is determined by sieving.

3. The formed cellulose article as claimed in claim 1, wherein the cellulosic matrix consists either of pure cellulose or of a mixture of more than 50 wt% cellulose with polymeric additives, preferably cellulose derivatives, and/or particulate additives, preferably carbon black, activated carbon, ion exchange resins, inorganic pigments and/or salts.

4. The formed cellulose article as claimed in claim 1, wherein the hexacyanoferrates comprise from 10 to 50 wt% of the overall weight thereof.

5. The formed cellulose article as claimed in claim 1, being a fibrid, a fiber, a fibrous nonwoven web, a granule, a bead, a self-supporting film, a tubular film, a filament, a sponge or a bristle.

6. The use of the formed cellulose article as claimed in one of more of claims 1 to 5 for sorbing monovalent metal ions, especially of the elements thallium and cesium and isotopes thereof, in aqueous or moist systems.

7. The use of a material as claimed in claim 6 for water treatment and water decontamination, metal beneficiation, wound treatment with wound dressings, air filtration, gas filtration and protective apparel.

## Revendications

1. Corps formé cellulosique en Lyocell destiné à lier des ions de métaux lourds et leurs isotopes radioactifs, **caractérisé en ce qu'**un ou plusieurs hexacyanoferrates sont intégrés dans une matrice cellulosique, complètement entourés par celle-ci et répartis de manière homogène sur toute la section transversale de la matrice cellulosique, dans lequel les hexacyanoferrates sont présents dans une proportion de 0,1 à 80 % en poids du poids total du corps formé cellulosique.

2. Corps formé cellulosique selon la revendication 1, **caractérisé en ce que** l'hexacyanoferrate est une liaison chimique neutre constituée d'anions d'hexacyanoferrates et de cations, de préférence du cobalt, du cuivre, du sodium, du potassium ou de l'ammonium, particulièrement de préférence du fer, avec des dimensions de particules comprises entre 0,001 µm et 100 µm, de préférence entre 0,1 µm et 50 µm, la dimension des particules étant déterminée par tamisage.

3. Corps formé cellulosique selon la revendication 1, dans lequel la matrice cellulosique est constituée soit de cellulose pure soit d'un mélange de plus de 50 % en poids de cellulose avec des additifs polymères, de préférence des dérivés de cellulose, et/ou des additifs particulaires, de préférence de la suie, du charbon actif, des résines échangeuses d'ions, des pigments anorganiques et/ou des sels.

4. Corps formé cellulosique selon la revendication 1, dans lequel les hexacyanoferrates sont présents dans une proportion de 10 à 50 % en poids du poids total.

5. Corps formé cellulosique selon la revendication 1, **caractérisé en ce qu'**il s'agit d'une fibride, d'une fibre, d'un non-tissé, d'un granulat, d'une perle, d'une feuille, d'un tuyau souple, d'un filament, d'une éponge ou d'une soie de brosserie.

6. Utilisation du corps formé cellulosique selon une ou plusieurs des revendications 1 à 5 en vue de la sorption d'ions métalliques monovalents, en particulier des éléments thallium et césium et de leurs isotopes, dans des systèmes aqueux ou humides.

7. Utilisation d'un matériau selon la revendication 6 afin de traiter et décontaminer de l'eau, d'extraire du métal, de traiter une plaie avec des compresses, de filtrer de l'air, de filtrer du gaz et de fabriquer des vêtements de protection.
